# EUROPEAN PATENT SPECIFICATION

(11) **EP 2 493 438 B1**
(45) Date of publication and mention of the grant of the patent: **22.11.2017**
(21) Application number: 10771296.0
(22) Date of filing: 19.10.2010
(51) Int. Cl.: A61F 9/00, A61B 3/113

(54) **OPHTHALMIC FLUID PUMP**
PUMPE FÜR OPHTHALMISCHE FLÜSSIGKEIT
POMPE À FLUIDE OPHTALMIQUE

(30) Priority: 29.10.2009 US 256111 P; 12.10.2010 US 902603
(43) Date of publication of application: 05.09.2012
(73) Proprietor: Johnson & Johnson Vision Care, Inc., Jacksonville, FL 32256 (US)
(72) Inventor: HALL, Gary S., Jacksonville, FL 32259 (US); MORLEY, Catie A., St. Johns, FL 32259 (US); VOSS, Leslie A., Jacksonville, FL 32258 (US)
(74) Representative: Tunstall, Christopher Stephen
(86) International application number: PCT/US2010/053144
(87) International publication number: WO 2011/053479

(56) References cited:
- EP-A2- 1 493 410
- WO-A1-2004/028421
- WO-A2-99/52479

## Description

### FIELD OF USE

This invention describes a device for dispensing liquids or mists into the eye, and, more specifically, in some embodiments, a device that dispenses a spray or mist into the eye based upon detection of a blink.

### BACKGROUND

It has been known to dispense a liquid or a mist into an eye using many different devices. However, although many devices result with a liquid entering the eye, the experience of getting the liquid into the eye is generally less than satisfactory.

Devices for self dispensing liquids typically require that a user hold the eyelids open to fight the blink reflex. This contention inhibits easy application of the desired fluids. Some automated devices pull down on one lid, or encapsulate the eye area to stop the lids from closing. This touch is damaging to makeup, and can lead to contamination of the device and the liquid entering the eye.

The dose from the system should consistently, without great user effort, dispense into the user's eye, not upon the eyelid or other part of the user's face, and optimally should not touch the face in a manner that damages makeup or contaminates the device.

Some dispensing devices simulate a 'gun' and shoot a fluid in at the eye at a rate calculated to beat the blink reflex, however the speed and impact of the fluid seem to induce discomfort in the patient.

Other devices force the lids open in different manners through touching the cheek below the lid, and the eyebrow range above the upper lid, then spray the fluid into the eye. This forcing open of the lids is uncomfortable, and the unit itself becomes large and unwieldy. Any makeup worn by the consumer is smudged during the process and sometimes contaminates the dispenser and/or the dispensed liquid.

Misting of fluid over the entire eye or even the facial area is also feasible, but wets not only the eye, but undesirable surfaces such as the eyelid, forehead, and nose. Application of liquid to the eyelid is declared to also wet the eye by flowing into the eye, but results from this method are mixed, and the wetting of the lid itself is typically undesirable.

International patent application WO 2004/028421A1 describes an apparatus (200) for treating an eye of a subject which comprises a device for sensing the open/closed state of an eye, an applicator (202) for delivering a substance to the eye, and a control system (106) that permits delivery of the substance when the sensing device detects that the eye is open but prevents delivery of the substance when the sensing device detects that the eye is closed.

### SUMMARY

Accordingly, the present invention includes an automated device with a pump, wherein the pump responds to an electrical signal to administer a dose of a liquid into an eye. In some embodiments, a microcontroller is used to control the pump. Some additional embodiments include a predetermined amount of liquid being dispensed. The predetermined amount may be according to a separate reservoir containing a single dose of liquid to be administered or via specific control of duration of a pump cycle which corresponds with a predetermined amount of liquid being pumped.

In some embodiments, the present invention automatically senses a blink or other external condition and dispenses the liquid in a timely fashion based upon the external condition. For example, a microcontroller may receive a signal that a blink has occurred and time administration of a liquid following the blink to allow the liquid to enter the eye before the eye may blink again. By using the window just after the blink, the present invention consistently provides enough time to dispense into the eye and also dispense at a rate which is an adequately slow application of fluid into the eye to maintain the inertial impact of the fluid on the eye at a comfortable level.

### DESCRIPTION OF THE DRAWINGS

FIG. 1 illustrates a dispensing device sensing a closed eye according to some embodiments of the present invention.
FIG. 2 illustrates a dispensing device sensing an open eye according to some embodiments of the present invention.
FIG. 3 illustrates an exemplary apparatus for sensing a state of an open or closed eye.
Fig. 4 illustrates a pump suitable for administering a predetermined dose of a liquid to an eye.
Fig. 5 illustrates a microcontroller that may be used to implement some embodiments of the present invention.

### DETAILED DESCRIPTION OF THE INVENTION

The present invention includes an automated device for administering a predetermined dose of a liquid into an eye. with the features of claim 1. The device includes a pump, wherein the pump responds to an electrical signal to administer a dose of a liquid into an eye. In some embodiments, a microcontroller is used to control the pump. A predetermined amount may be administered according to a command signal controlled by the microcontroller. The control signal may, for example, determine a voltage supplied to the pump, wherein the voltage causes one or more of: a specific rate of pumping, a specific period of pumping and a particular reservoir drawn from during a pumping action.

In some embodiments, the predetermined amount may be according to a separate reservoir containing a single dose of liquid to be administered or via specific control of a duration of a pump cycle which corresponds with a predetermined amount of liquid being pumped.

In some embodiments with a blink detector, the device for dispensing a liquid or a mist into an eye includes a detection mechanism to determine when a blink has been completed. Dispensing is timed to an interval based upon a determination of when an eye into which the liquid will be dispensed opens and closes, such as, for example, in a consciously induced eye blink or a natural eye blink. The blink is utilized to determine whether an eye is known to be open whereby a liquid can be dispensed before the patient closes the eye.

Microcontroller: (also sometimes referred to as a microcontroller unit, MCU or µC) as used herein refers to an integrated circuit consisting of a central processing unit (CPU) combined with support function circuitry such as a crystal oscillator, timers, watchdog timer, serial and analog I/O etc., program memory in the form of NOR flash or OTP ROM is also often included, as well as some amount of RAM. Some suitable microcontrollers may operate at clock rate frequencies as low as 4 kHz, as this is adequate for many typical applications, enabling low power consumption (milliwatts or microwatts). They will generally have the ability to retain functionality while waiting for an event such as a change state microcontroller or other interrupt.

In some embodiments the device includes features to minimize the need for facial contact during liquid application. Additional embodiments include alignment features to assure proper alignment of the device to the eye. For example, in some embodiments, the dispensing device includes protrusions that rest on the eyebrow, and have a small diameter hole for the user to look through. When the user is able to see through the hole, proper alignment has been achieved).

Once the device is properly aligned with an eye, opening and closing of the eye is automatically determined by a sensor. A dispensing apparatus in logical communication with the sensor is programmed to dispense a liquid or mist into the eye according to the timing of an open cycle of a blink. The alignment process coupled with the blink detection eliminates the need for holding the lids open, touching the face, or contamination associated with regular facial touch. In addition, by dispensing based upon an opening motion of an eyelid, a dispenser according to the present invention, consistently wets the eye without wetting the eyelid or surrounding face.

In the following sections detailed descriptions of embodiments of the invention will be given. The description of both preferred and alternative embodiments are exemplary embodiments only, and it is understood that to those skilled in the art that variations, modifications and alterations may be apparent. It is therefore to be understood that said exemplary embodiments do not limit the scope of the underlying invention.

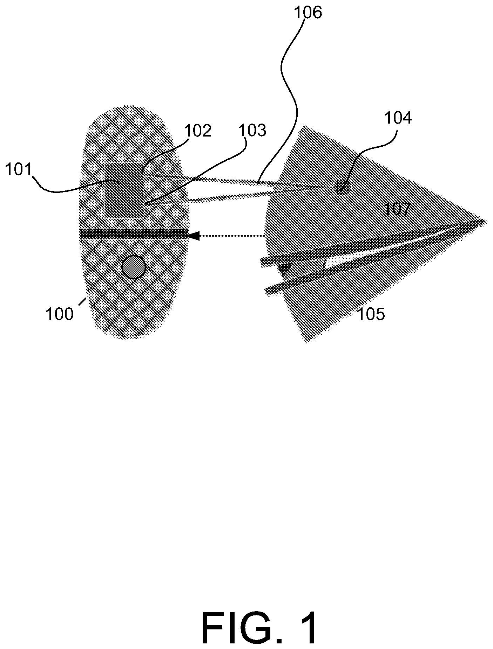

Referring now to Fig. 1, in some embodiments, a liquid dispensing device 100 includes a sensor 101 capable of sensing an open state or a closed state of an eye 105. In some embodiments, the sensor 101 includes an emitter 102 and a detector 103. The emitter 102 emits a beam 106 which reflects off of a reflecting point 104 and back to the detector 103. As illustrated in Fig. 1, the reflecting point 104 is on the eyelid 107 of the eye. The beam 106 may include, for example one or more of: infra red light, visible light, ultrasonic wavelengths, or other wavelengths.

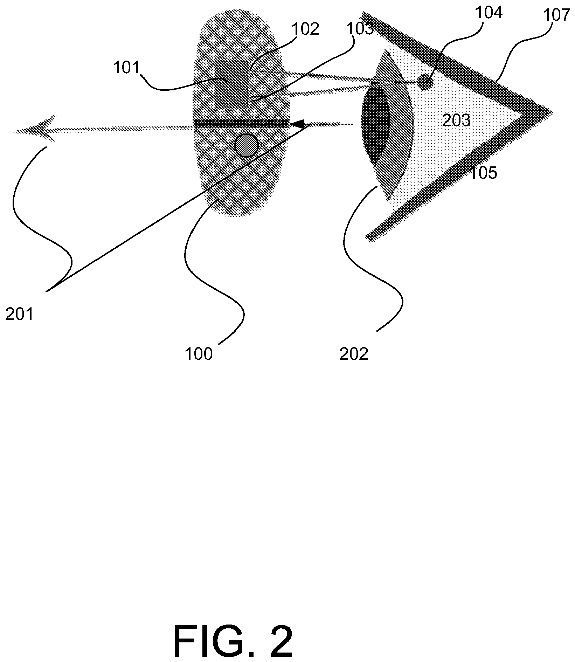

Referring now to Fig. 2, an open eye 105 provides for a reflecting point 104 on an open portion 203 of the eye 105, as opposed to the eyelid 107. The open portion of the eye 203 can include, for example, reflection off of the sclera or other portion of the eye. In some embodiments, a wavelength of an emitted beam is correlated with physical characteristics of the reflecting point 104. Reflection of the emitter beam 106 off of the eyelid will reflect back with a first set of reflection characteristics and reflection off of the open portion of the eye 203 with a second set of reflection characteristics. The reflection characteristics will be sensed by the detector 103.

In another aspect, of the present invention, alignment of the eye 105 with the liquid dispensing device 100 is facilitated by the liquid dispensing device. According to the present invention, a focal point of the emitter 102 is aligned with the pupil of the eye. Alignment is accomplished via a line of sight 201. In some embodiments, when a pupil 202 is aligned with the line of sight 201, the sensor 101 is also properly aligned to sense an open state and a closed state of the eye 105. For example, in some embodiments, when a patient aligns to a tubular cutout comprising a line of sight 201 in the dispensing device, it creates a coaxial alignment between the line of sight and the center of the tube. The alignment establishes an angular and X-Y location of the pupil relative to the dispensing device 100, wherein X-Y correlate to a vertical and horizontal planar position at a given distance from the eye.

In some embodiments, a line of sight can be combined with a positioning device which includes one or more alignment legs which press against the face and/or forehead.

In addition, in some embodiments, a sensor measures the distance of the device from the eye and signals (perhaps by click, sound, or vibration) that it is within an acceptable Z positional range from the eye for an optimal dose wherein the Z dimension correlates with a depth or distance from the eye. It is expected that this range will be relatively wide (in the 2-5 mm range) so any of the sensors noted above as able to detect the blink could also be used to detect a distance from the eye to the dispensing device 100.

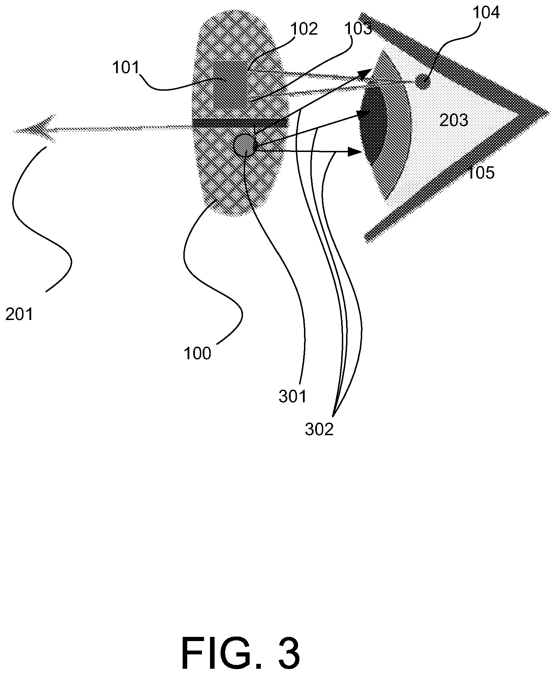

Referring now to Fig. 3, a dispensing nozzle 301 will dispense a liquid via sprays 302 or mist (not illustrated) when the sensor 101 senses that the eye 105 is in an open state. The open state is determined by the nature of the beam 106 sensed by the detector 103. Preferred embodiments, dispense a liquid spray 302 based upon timing that indicates that the eyelid 107 is involved in an opening cycle. Dispensing sprays 302 during an opening cycle of an eye can be accomplished such that the patient cannot physically blink to close the eye before the liquid is dispensed. The spray may be a liquid stream or a mist.

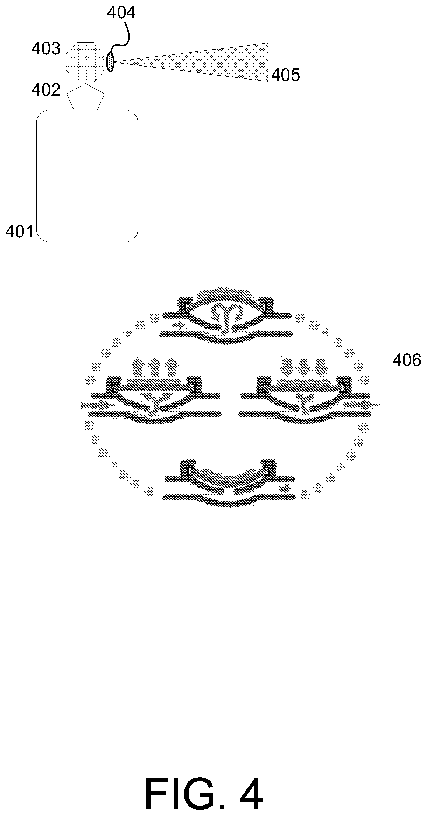

Referring now to Fig. 4, an exemplary pumping device illustrated with a main reservoir 401, a dosing reservoir 402, an electrically controlled pumping mechanism 403 and an eye orifice nozzle 404. When the pumping mechanism 403 is activated it draws from one or both of the main reservoir 401 and the dosing reservoir 402 and pumps a liquid spray 405 or liquid mist into an eye proximate to the eye orifice nozzle 404.

The main reservoir 401 can contain a liquid to be dispensed into the eye. The liquid can include, for example, a solution useful for treating dryness or other condition in the eye, a medicament, a nutrient or other substance efficacious to the eye.

In some embodiments, a single dose reservoir 402 is included. Other embodiments work directly from the main reservoir 401. The single dose reservoir 402 is in liquid communication with the main reservoir 401 and can be filled with an amount generally equal to a single dose of liquid to be administered to the eye.

The pump draws from one or both of the single dose reservoir 402 and the main reservoir 401. The pump can include a piezo electric pump, a diaphragm type pump, a positive displacement type pump or other device capable of pumping specific amounts of a liquid into the eye. In some preferred embodiments, a piezo electric type pump generally used to administer specific amounts of liquid on a regular basis, such as for example, a piezo electric pump used to pump pharmaceuticals into a an intravenous feed, may be adapted to pump a liquid from one or both of the reservoirs 401-402, through the eye nozzle orifice. Typically, a pharmacy administering pump would need to be adapted to pump with a shorter duration and higher pressure action in order to provide the pulsatile delivery required to administer a liquid to an eye. This is a change from the constant low speed, but tightly controlled amounts fed into an intravenous feed. Some specific examples can include the MP5 and MP6 offered by Bartels Mikrotechnik GmbH. A functional diagram of how a micropump may operate is also included 406.

Specifications may include, for example:
Pump type piezoelectric diaphragm pump
Number of actuators 2
Dimensions without connectors 30 x 15 x 3.8 mm³
Weight 2 g
Fluidic connectors tube clip (outer diameter 1.6 mm, length 3.5 mm)
Electric connector flex connector / Molex FCC
1:25 mm pitch
Power consumption < 200 mW
Self-priming yes 2
Pumping media liquids, gases and mixtures
Operating temperature 0 - 70°C 3
Life time 5000 h 3
IP code IP33 4
Materials in contact with media polyphenylene sulphone (PPSU)
Suitable evaluation controller mp-x and mp6-OEM
Typical values of flow and back pressure for selected media
(values defined with mp-x: 250 V, SRS):
Gases Max. flow on request
Max. back pressure on request
Liquids Water Max. flow 6 ml/min +/- 15% (100 Hz)
   Max. back pressure 550 mbar +/- 15% (100 Hz)

Additional examples of pump specifications may include:
Pump type piezoelectric diaphragm pump
Number of actuators 1
Dimensions without connectors 14 x 14 x 3.5 mm³
Weight 0.8 g
Fluidic connectors tube clip (outer diameter 2 mm,
length 3 mm)
Electric connector flex connector / phone jack
Power consumption < 200 mW
Self-priming yes 2
Pumping media liquids or gases
Operating temperature 0 - 70°C
Life time 5000 h 3
IP code IP44
Materials in contact with media polyphenylene sulphone (PPSU),
polyimide (PI), nitrile butadiene
rubber (NBR)
Suitable evaluation controller mp-x and mp5-a
Typical values of flow and back pressure for selected media
(values defined with mp-x: 250 V, SRS):
Gases Max. flow 15 ml/min (300 Hz)
Linear range 0 - 5 ml/min @ 0 - 50 Hz
Max. back pressure 30 mbar (300 Hz)
Liquids Water Max. flow 5 ml/min (100 Hz)
Linear range 0 - 3 ml/min @ 0 - 30 Hz
Max. back pressure 250 mbar (100 Hz)
Repeatability
(30 Hz, 250 V, SRS)
< 12 %
Viscosity <~ 120 mPas
1 Typical values. Values can vary under application conditions. Content is subject to
changes without notice. 2
Conditions: suction pressure < 10 mbar, DI water, settings mp-x: 100 Hz, 250 V, SRS, the max. flow rate can be reached by manual priming.
   3 Conditions: DI water, room temperature, settings mp-x: 100 Hz, 250 V, SRS.

In some preferred embodiments, the pump will provide enough pressure to spray into an eye from a distance of about 15 millimeters (mm). Distances may therefore be between about 5 mm and 45 mm. In addition, an amount of spray should be controllable within about 5 micro liters of accuracy in amounts of between 3 and 30 micro liters, and preferably about 15 micro liters.

According to some embodiments of the present invention, the detector is placed in logical communication with the automated pump 403 capable of dispensing a predetermined amount of a liquid through the eye orifice nozzle and into the eye.

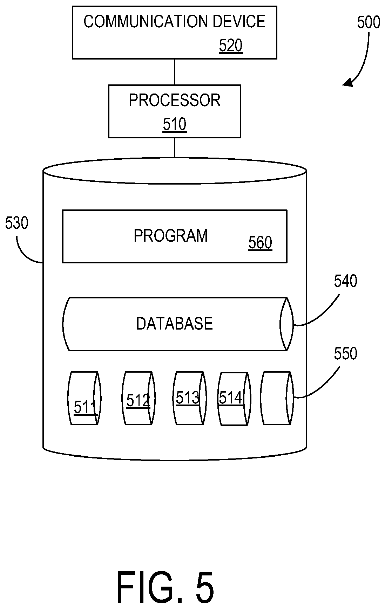

Referring now to Fig. 5 a microcontroller 500 is illustrated that may be used in some embodiments of the present invention. The microcontroller 500 includes a processor 510, and one or more processor components and/or support function circuitry 511-514 such as a crystal oscillator, timers, watchdog timer, serial and analog I/O etc.; program memory in the form of NOR flash or OTP ROM is also often included, as well as some amount of RAM.

The microcontroller 500 may also include a communication device 520. In some embodiments, a microcontroller 500 can be used to receive a logical indication that an eye is in a first state or a second state and transmit energy to a liquid dispenser at a time appropriate to dispense a liquid or mist into the eye, based upon the transition from a first state to a second state. Other logic may also be programmed into the microcontroller and provide for flexibility of function. By way of non-limiting example, such functionality may include monitoring how much fluid is currently stored in one or both of the main reservoir and the dose reservoir; duration of pump actuation which correlates into an amount of liquid administered to the eye, which reservoir is being drawn from; periodic timing of liquid disbursement; duration of liquid disbursement and almost any other functionality related to the operation of the pump.

The one or more processors can be coupled to a communication device 520 configured to communicate energy via a communication channel. The communication device may be used to electronically control, for example, one or more of: timing of liquid dispensing; an amount of liquid dispensed; a duration of a dispensing motion; tracking a number of dispensing actions; tracking chronological dispensing patterns or other actions related to the dispensing.

The processor 510 is also in communication with a storage device 530. The storage device 530 may comprise any appropriate information storage device, including for example: semiconductor memory devices such as Random Access Memory (RAM) devices and Read Only Memory (ROM) devices.

The storage device 530 can store a program 560 for controlling the processor 510. The processor 510 performs instructions of the program 560, and thereby operates in accordance with the present invention. For example, the processor 510 may receive information descriptive of liquid to be dispensed, dispensing amounts, dispensing patterns, and the like.

In addition, the present invention may include an Energy Source 550, such as an electrochemical cell or battery as the storage means for the energy and in some embodiments, encapsulation and isolation of the materials comprising the Energy Source from an environment into which an ophthalmic pump is placed. The Energy Source 550 can provide power to activate the microcontroller. In some embodiments, power consumption of a microcontroller while sleeping (CPU clock and most peripherals off) may be just nanowatts.

### Conclusion

The present invention, as described above and as further defined by the claims below, provides an apparatus as described in claim 1.

## Claims

1. An apparatus (100) for dispensing a liquid in an eye, the apparatus comprising:
one or more reservoirs (401, 402);
an automated pump (403) capable of being actuated based upon receipt of a control signal, said pump in liquid communication with one or more reservoirs (401, 402);
a signal generator (520) for generating a signal to actuate the automated pump (403); and
an eye orifice nozzle (301) through which the automated pump (403) will pump liquid contained in the one or more reservoirs (401, 402) based upon a signal generated by the signal generator (520);
**characterised in that** the apparatus further comprises an alignment apparatus for aligning an eye with the eye orifice nozzle (301), wherein the alignment apparatus is configured to align the pupil of the eye with a line of sight (201).

2. The apparatus of claim 1 additionally comprising a sensor (101), which when placed in position proximate to an eye is functional to detect a first state of an eye and a second state of an eye and generate a signal indicating that the eye is in one of said first state and said second state eye.

3. The apparatus of claim 2 wherein the sensor (101) comprises an emitter (102) emitting a signal in a predetermined wavelength.

4. The apparatus of claim 3 wherein the sensor additionally comprises a detector (103) capable of detecting a signal in the predetermined wavelength.

5. The apparatus of claim 4 wherein the sensor (101) generates the signal indicating if the eye is in the first state or the second state.

6. The apparatus of claim 5 additionally comprising a microcontroller (500) for receiving the signal indicating if the eye is in the first state or the second state and generating a control signal to the pump (403) to operate the pump (403) based upon the signal indicating if the eye is in the first state or the second state.

7. The apparatus of claim 1 wherein the alignment apparatus additionally comprises an alignment fixture.

8. The apparatus of claim 6 additionally comprising a dose reservoir (402) capable of storing a predetermined amount of a liquid to be administered into an eye.

9. The apparatus of claim 6 wherein the microcontroller (500) comprises a storage device (530) storing executable code for controlling the pump (403) to dispense a predetermined dosing amount based upon the indication of the first state or second state of the eye.

10. The apparatus of claim 9 wherein the microcontroller (500) comprises a storage device (530) storing executable code for tracking a number of doses administered during a time period.

11. The apparatus of claim 6 wherein the microcontroller (500) comprises a storage device (530) storing executable code for operating the pump (403) for a predetermined period of time based upon a signal indicating an external condition.

12. The apparatus of claim 11 wherein the external condition comprises a movement of an eye to an open position.

## Patentansprüche

1. Vorrichtung (100) zum Ausgeben einer Flüssigkeit in ein Auge, wobei die Vorrichtung umfasst:
ein oder mehrere Resevoirs (401, 402);
eine automatisierte Pumpe (403), die fähig ist, auf der Grundlage des Empfangens eines Steuersignals betätigt zu werden, wobei die Pumpe in Flüssigkeitsverbindung mit einem oder mehreren Resevoirs (401, 402) steht;
einen Signalgenerator (520) zum Erzeugen eines Signals zum Betätigen der automatisierten Pumpe (403); und
eine Augenöffnungsdüse (301), durch die die automatisierte Pumpe (403) auf der Grundlage eines von dem Signalgenerator (520) erzeugten Signals Flüssigkeit, die in dem einen oder den mehreren Resevoirs (401, 402) enthalten ist, pumpen wird;
**dadurch gekennzeichnet, dass** die Vorrichtung ferner eine Ausrichtungsvorrichtung zum Ausrichten eines Auges mit der Augenöffnungsdüse (301) umfasst, wobei die Ausrichtungsvorrichtung dafür gestaltet ist, die Pupille des Auges mit einer Sichtlinie (201) auszurichten.

2. Vorrichtung gemäß Anspruch 1, zusätzlich umfassend einen Sensor (101), der, wenn in einer Position nahe einem Auge angeordnet, funktionsfähig ist, einen ersten Zustand eines Auges und einen zweiten Zustand eines Auges zu erfassen und ein Signal zu erzeugen, um anzuzeigen, dass sich das Auge in einem von dem ersten Zustand und dem zweiten Zustand des Auges befindet.

3. Vorrichtung gemäß Anspruch 2, wobei der Sensor (101) einem Emitter (102) umfasst, der ein Signal mit einer vorbestimmten Wellenlänge emittiert.

4. Vorrichtung gemäß Anspruch 3, wobei der Sensor zusätzlich einen Detektor (103) umfasst, der fähig ist, ein Signal mit der vorbestimmten Wellenlänge zu erfassen.

5. Vorrichtung gemäß Anspruch 4, wobei der Sensor (101) das Signal erzeugt, das anzeigt, ob sich das Auge in dem ersten Zustand oder dem zweiten Zustand befindet.

6. Vorrichtung gemäß Anspruch 5, zusätzlich umfassend eine Mikrosteuerung (500) zum Empfangen des Signals, das anzeigt, ob sich das Auge in dem ersten Zustand oder dem zweiten Zustand befindet, und Erzeugen eines Steuersignals für die Pumpe (403), um die Pumpe (403) auf der Grundlage des Signals, das anzeigt, ob sich das Auge in dem ersten Zustand oder dem zweiten Zustand befindet, zu betreiben.

7. Vorrichtung gemäß Anspruch 1, wobei die Ausrichtungsvorrichtung zusätzlich eine Ausrichtungsfixierung umfasst.

8. Vorrichtung gemäß Anspruch 6 zusätzlich einem Dosisreservoir (402), das fähig ist, eine vorbestimmte Menge einer in ein Auge zu verabreichenden Flüssigkeit aufzubewahren.

9. Vorrichtung gemäß Anspruch 6, wobei die Mikrosteuerung (500) eine Speichervorrichtung (530) umfasst, die einen ausführbaren Code zum Steuern der Pumpe (403) zum Ausgeben einer vorbestimmten Dosismenge auf der Grundlage der Anzeige des ersten Zustands oder des zweiten Zustands des Auges speichert.

10. Vorrichtung gemäß Anspruch 9, wobei die Mikrosteuerung (500) eine Speichervorrichtung (530) umfasst, die einen ausführbaren Code zum Verfolgen der Zahl von während eines Zeitraums verabreichten Dosen speichert.

11. Vorrichtung gemäß Anspruch 6, wobei die Mikrosteuerung (500) eine Speichervorrichtung (530) umfasst, die einen ausführbaren Code zum Betreiben der Pumpe (403) für eine vorbestimmte Zeitdauer auf der Grundlage eines Signals, das einen externen Zustand anzeigt, speichert.

12. Vorrichtung gemäß Anspruch 11, wobei der externe Zustand eine Bewegung eines Auges zu einer offenen Stellung umfasst.

## Revendications

1. Appareil (100) de dispense d'un liquide dans un oeil, l'appareil comprenant :
un ou plusieurs réservoirs (401, 402) ;
une pompe automatisée (403) capable d'être actionnée sur la base de la réception d'un signal de commande, ladite pompe étant en communication liquide avec un ou plusieurs réservoirs (401, 402) ;
un générateur de signal (520) pour générer un signal afin d'actionner la pompe automatisée (403) ; et
une buse à orifice oculaire (301) à travers laquelle la pompe automatisée (403) pompera un liquide contenu dans les un ou plusieurs réservoirs (401, 402) sur la base d'un signal généré par le générateur de signal (520) ;
**caractérisé en ce que** l'appareil comprend en outre un appareil d'alignement pour aligner un oeil avec la buse à orifice oculaire (301), l'appareil d'alignement étant configuré pour aligner la pupille de l'oeil avec une ligne de visée (201).

2. Appareil selon la revendication 1, comprenant de surcroît un capteur (101) qui, lorsqu'il est placé dans une position à proximité d'un oeil, est fonctionnel pour détecter un premier état d'un oeil et un second état d'un oeil et générer un signal indiquant que l'oeil est dans l'un dudit premier état et dudit second état de l'oeil.

3. Appareil selon la revendication 2, dans lequel le capteur (101) comprend un émetteur (102) émettant un signal à une longueur d'onde prédéterminée.

4. Appareil selon la revendication 3, dans lequel le capteur comprend de surcroît un détecteur (103) capable de détecter un signal à la longueur d'onde prédéterminée.

5. Appareil selon la revendication 4, dans lequel le capteur (101) génère le signal indiquant si l'oeil est dans le premier état ou le second état.

6. Appareil selon la revendication 5, comprenant de surcroît une micro-unité de commande (500) pour recevoir le signal indiquant si l'oeil est dans le premier état ou le second état et générer un signal de commande en direction de la pompe (403) pour actionner la pompe (403) sur la base du signal indiquant si l'oeil est dans le premier état ou le second état.

7. Appareil selon la revendication 1, dans lequel l'appareil d'alignement comprend de surcroît un gabarit d'alignement.

8. Appareil selon la revendication 6, de surcroît un réservoir de dose (402) capable de stocker une quantité prédéterminée d'un liquide à administrer dans un oeil.

9. Appareil selon la revendication 6, dans lequel la micro-unité de commande (500) comprend un dispositif de stockage (530) stockant un code exécutable pour commander à la pompe (403) de dispenser une quantité de dosage prédéterminée sur la base de l'indication du premier état ou du second état de l'oeil.

10. Appareil selon la revendication 9, dans lequel la micro-unité de commande (500) comprend un dispositif de stockage (530) stockant un code exécutable pour le suivi d'un nombre de doses administrées pendant une période de temps.

11. Appareil selon la revendication 6, dans lequel la micro-unité de commande (500) comprend un dispositif de stockage (530) stockant un code exécutable pour actionner la pompe (403) pendant une période de temps prédéterminée sur la base d'un signal indiquant une condition externe.

12. Appareil selon la revendication 11, dans lequel la condition externe comprend un mouvement d'un oeil vers une position ouverte.
